# DEMANDE DE BREVET EUROPEEN

(11) **EP 1 297 823 A2**
(43) Date de publication de la demande: **02.04.2003**
(21) Numéro de dépôt: 02291417.0
(22) Date de dépôt: 07.06.2002
(51) Int. Cl.: A61K 7/48

(54) **Composition cosmétique et/ou dermatologique stabilisée**

(30) Priorité: 11.06.2001 FR 0107609
(71) Demandeur: L'OREAL, 75008 Paris (FR)
(72) Inventeur: L'Alloret, Florence, 75013 Paris (FR); Aubrun-Sonneville, Odile, 92160 Antony (FR)
(74) Mandataire: Rasson, Catherine

(57) **Abrégé**

La composition sous forme d'émulsion eau dans huile comprenant une phase aqueuse dans une phase huileuse, est caractérisée en ce qu'elle comprend une quantité stabilisatrice de la phase aqueuse, d'au moins un polymère ou copolymère amphiphile, de l'acide 2-acrylamido-2-méthylpropane sulfonique portant des greffons hydrophobes choisis parmi les radicaux hydrocarbonés ayant de 6 à 30 atomes de carbone, les radicaux hydrocarbonés oxyalkylénés comprenant un groupe hydrocarboné ayant de 6 à 30 atomes de carbone et au moins un motif oxyalkylène, et leurs mélanges.

## Description

La présente invention concerne, de manière générale, une composition cosmétique et/ou dermatologique se présentant sous la forme d'une émulsion eau-dans-huile, de préférence dépourvue de tensioactif émulsionnant ou contenant un faible taux de tensioactif émulsionnant, comprenant une phase aqueuse stabilisée dans une phase huileuse à l'aide d'un polymère ou copolymère amphiphile d'acide 2-acrylamido-2-méthylpropane sulfonique, liposoluble ou lipodispersible ou soluble ou dispersible dans un solvant polaire.

Les émulsions eau-dans-huile sont des dispersions de globules aqueux dans une phase continue huileuse. Ces émulsions sont en général stabilisées par des molécules amphiphiles de faible masse molaire (< 5000 g/mole) tels que les composés alkylglycérolés, les composés alkylpolyoxyéthylénés ou encore des composés siliconés. Ces émulsions présentent les inconvénients suivants :
- l'utilisation de tensioactifs émulsionnants relativement agressifs vis-à-vis de la peau, et présents à des concentrations supérieures à 1% (en poids) ;
- leur stabilité est difficile à obtenir car les forces répulsives entre gouttes, d'origine stérique, sont de faible portée. Ceci se traduit par un phénomène de floculation, qui peut engendrer de la sédimentation et/ou encore de la coalescence ;
- les composés amphiphiles siliconés sont spécifiques aux huiles de même nature, ce qui limite la gamme d'huiles utilisables.

Il existe également des polymères siliconés permettant de stabiliser des émulsions eau-dans-huile ; la phase huileuse doit dans ce cas contenir en majorité une huile siliconée.

La demande de brevet EP-A-1,069,142 concerne des polymères hydrosolubles obtenus par polymérisation radicalaire d'au moins un macromonomère comprenant un bloc hydrophile polyoxyalkyléné et un bloc hydrophobe pouvant comporter de 1 à 30 atomes de carbone avec au moins un comonomère oléfiniquement insaturé comportant de l'oxygène, de l'azote, du soufre, du phosphore, du chlore et/ou du fluor, en particulier l'acide acrylamidométhylpropane sulfonique. Ces polymères peuvent être utilisés en tant qu'épaississant, émulsionnant, dispersant, agent de suspension, stabilisant et/ou agent de consistance pour préparation aqueuse, notamment dans les domaines cosmétique et/ou pharmaceutique.

Il existe donc le besoin d'une composition stable se présentant sous la forme d'une émulsion eau dans huile (E/H), utilisable notamment dans les domaines cosmétique et/ou dermatologique, qui :
- contient de faibles teneurs de tensioactif émulsionnant (c'est-à-dire contient 1% ou moins en poids de tensioactif émulsionnant par rapport au poids total de la composition), de préférence moins de 1% en poids et mieux n'en contient pas du tout ;
- peut contenir des huiles hydrocarbonées en quantités importantes (c'est-à-dire 5% ou plus en poids par rapport au poids total de la composition) ; et
- soit stable vis-à-vis de la floculation.

La demanderesse a découvert de façon surprenante que les polymères et copolymères dérivés de l'acide 2-acrylamido-2-méthylpropane sulfonique (AMPS) et portant des greffons hydrophobes choisis parmi les radicaux hydrocarbonés en C₆-C₃₀, les radicaux hydrocarbonés en C₆-C₃₀ comportant au moins un motif oxyalkylène, les radicaux perfluoroalkyles, les radicaux siliconés, et leurs mélanges, permettent d'obtenir des émulsions eau dans huile qui ne floculent pas et peuvent contenir des huiles hydrocarbonées en quantités importantes. Le taux de greffage molaire en motif hydrophobe des polymères est supérieur ou égal à 50 %, de préférence supérieur ou égal à 60 % et mieux encore 65 %.

Ainsi, l'invention a pour objet une composition cosmétique et/ou dermatologique sous forme d'une émulsion eau-dans-huile comprenant une phase aqueuse dispersée dans une phase huileuse et une quantité stabilisatrice de la phase aqueuse d'au moins un polymère ou copolymère amphiphile de l'acide 2-acrylamido-2-méthylpropane sulfonique portant des greffons hydrophobes choisis parmi les radicaux hydrocarbonés ayant de 6 à 30 atomes de carbone, les radicaux hydrocarbonés oxyalkylènés comprenant un groupe hydrocarboné ayant 6 à 30 atomes de carbone et au moins un motif oxyalkylène, les radicaux perfluoroalkyles, les radicaux siliconés, et leurs mélanges, le taux de greffage molaire en motif hydrophobe dans le polymère ou copolymère étant au moins égal à 50 %.

L'invention a également pour objet l'utilisation dans une composition cosmétique et/ou dermatologique, sous forme d'une émulsion eau-dans-huile comprenant une phase aqueuse dispersée dans une phase huileuse, en tant qu'agent de stabilisation de la phase aqueuse, d'une quantité stabilisatrice d'au moins un polymère ou copolymère amphiphile de l'acide 2-acrylamido-2-méthylpropane sulfonique portant des greffons hydrophobes choisis parmi les radicaux hydrocarbonés ayant de 6 à 30 atomes de carbone, les radicaux hydrocarbonés oxyalkylénés comprenant un groupe hydrocarboné en C₆-C₃₀ et au moins un motif oxyalkylène, les radicaux perfluoroalkyles, les radicaux siliconés, et leurs mélanges, le taux de greffage molaire en motif hydrophobe du polymère ou copolymère étant au moins égal à 50 %.

Les polymères de l'invention sont des dérivés liposolubles ou lipodispersibles, ou solubles ou dispersibles dans un solvant polaire de l'acide 2-acrylamido-2-méthylpropane sulfonique (AMPS), portant des greffons alkyles et/ou alkylpolyoxyalkylénés et/ou perfluoroalkyles et/ou siliconés. Le taux de greffage molaire de ces greffons est supérieur ou égal à 50 % (c'est-à-dire 50% ou plus), de préférence supérieur ou égal à 60 % (60% ou plus), et mieux encore supérieur ou égal à 65 % (65 % ou plus).

Par polymères et copolymères liposolubles ou lipodispersibles, on entend des polymères et copolymères qui, introduits dans une huile ou un mélange huileux, à une concentration égale à 1% en poids, sous agitation à 50°C pendant 48 heures, conduisent à une solution macroscopiquement homogène dont la transmittance de la lumière, à une longueur d'onde égale à 500 nm, à travers un échantillon de 1 cm d'épaisseur, est d'au moins 50%, de préférence d'au moins 60%. L'huile considérée peut être de type alcane, ester, triglycéride, éther, siliconée, fluorée ou un mélange des huiles précédemment citées.

Par polymères et copolymères solubles ou dispersibles dans un solvant polaire, on entend des polymères et copolymères qui, introduits dans un solvant polaire à une concentration égale à 1% en poids, sous agitation à 50°C pendant 48 heures, conduisent à une solution macroscopiquement homogène. On entend ici par "solvant polaire" un solvant ayant une constante diélectrique mesurée à 25°C, inférieure à 50. Ce solvant peut être notamment choisi parmi les alcools inférieurs comportant 1 à 6 atomes de carbone, tels que l'éthanol (constante diélectrique : 24,6) ; les cétones telles que l'acétone (constante diélectrique : 20,7) ; les polyols tels que le propylène glycol (constante diélectrique : 30,2) ; les éthers tels que le diéthyléther (constante diélectrique : 4,3) ; et leurs mélanges.

Les polymères et copolymères conformes à l'invention sont des polymères et copolymères amphiphiles d'acide 2-acrylamido 2-méthylpropane sulfonique (AMPS).

On entend par polymère et copolymère amphiphile, tout polymère ou copolymère comportant à la fois une partie hydrophile et une partie hydrophobe.

La partie hydrophobe présente dans les polymères et copolymères de l'invention correspond aux chaînes grasses latérales ou greffons hydrophobes.

Les greffons hydrophobes sont liés au squelette principal du polymère ou copolymère de manière covalente, de préférence par l'intermédiaire d'un groupe de liaison fonctionnel, par exemple un groupe ester, amide, éther ou uréthane.

Les greffons hydrophobes sont choisis parmi les radicaux hydrocarbonés en C₆-C₃₀, de préférence en C₆-C₂₂, mieux en C₁₂-C₁₈, les radicaux hydrocarbonés définis précédemment et comportant en outre au moins un motif oxyalkylène, généralement 1 à 100 motifs oxyalkylène, mieux 1 à 30 motifs oxyalkylène, mieux encore de 3 à 20 motifs oxyalkylène, en particulier des motifs oxyéthylène, oxypropylène, les radicaux siliconés, les radicaux perfluoroalkyles et des mélanges de ces motifs.

Parmi les radicaux hydrocarbonés convenant pour les greffons hydrophobes selon l'invention, on peut citer :
- les radicaux alkyles, linéaires ou ramifiés, par exemple n-hexyle, n-octyle, n-décyle, n-hexadécyle, n-dodécyle, isostéaryle ;
- les radicaux alcényles, linéaires ou ramifiés, par exemple oléyle ;
- les radicaux alicylcliques, par exemple cyclododécane et adamantine ;

- le radical cholestéryle et les restes d'esters de cholestérol, par exemple le groupe oxyhexanoate de cholestéryle ;
- les radicaux polycycliques aromatiques, par exemple les radicaux naphtalényle et pyrenyle.

Comme radicaux perfluoroalkyles, on peut citer en particulier ceux en C₆-C₁₈, par exemple le radical de formule :

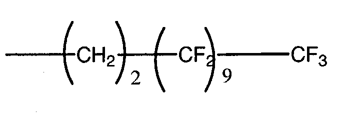

Comme radicaux siliconés, on peut utiliser notamment les polydiméthylsiloxanes (PDMS) de masse molaire inférieure à 20 000 g/mole.

Les greffons hydrophobes préférés sont les radicaux alkyles linéaires ou ramifiés, tels que les radicaux n-dodécyle, n-hexadécyle et isostéaryle, ou les radicaux alcényles tels que le radical oléyle.

Parmi les radicaux hydrocarbonés comportant 1 à 100 motifs oxyalkylène, on peut citer les radicaux de formule : 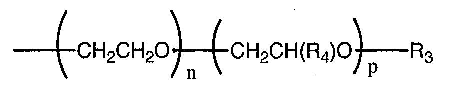 dans laquelle R₃ représente un radical hydrocarboné en C₆-C₃₀ tel que défini précédemment, R₄ représente l'hydrogène ou un groupe alkyle linéaire ou ramifié en C₁-C₆ (de préférence méthyle) et n et p sont, indépendamment l'un de l'autre, des entiers de 0 à 100, de préférence 1 à 30, mieux de 3 à 20 sous réserve que la somme n+p, non nulle, soit égale ou inférieure à 100, de préférence inférieure à 30 et mieux inférieure à 20.

Les polymères et copolymères conformes à l'invention de façon préférentielle sont neutralisés partiellement ou totalement par une base minérale (par exemple soude, potasse, ammoniaque, ammonium substitué par 1 à 4 groupes alkyle portant de 1 à 15 atomes de carbone) ou une base organique telle que la mono-, di- et tri-éthanolamine, un aminométhylpropanediol, la N-méthyl-glucamine, les acides aminés basiques comme l'arginine et la lysine, et leurs mélanges.

Les polymères et copolymères conformes à l'invention ont en général une masse molaire moyenne en poids allant de 50.000 à 10.000.000 g/mole, plus préférentiellement de 100.000 à 8.000.000 g/mole et encore plus préférentiellement de 100.000 à 7.000.000 g/mole.

Les polymères et copolymères amphiphiles d'AMPS selon l'invention peuvent être réticulés ou non-réticulés. Ils sont de préférence non-réticulés.

Les agents de réticulation peuvent être choisis parmi les composés à polyinsaturation oléfinique couramment utilisés pour la réticulation des polymères obtenus par polymérisation radicalaire. On peut citer par exemple le divinylbenzène, l'éther diallylique, le dipropylèneglycol-diallyléther, les polyglycol-diallyléthers, le triéthylèneglycol-divinyléther, l'hydroquinone-diallyléther, le tétrallyl-oxéthanoyle ou d'autres allyl-ou vinyléthers alcools polyfonctionnels, le diacrylate de tétraéthylèneglycol, la triallylamine, le triméthylolpropane-diallyléther, le tétraalloxyéthane, le méthylène-bis-acrylamide, les éthers allyliques d'alcools de la série des sucres, le méthylène-bis-acrylamide, le méthacrylate d'allyle, le triméthylol propane triacrylate (TMPTA) ou leurs mélanges.

On utilisera plus particulièrement le méthylène-bis-acrylamide, le méthacrylate d'allyle ou le triméthylol propane triacrylate (TMPTA). Le taux de réticulation varie de préférence de 0,01 à 10 % en moles et plus particulièrement de 0,2 à 2 % en moles par rapport au polymère ou copolymère.

Les polymères conformes à l'invention peuvent notamment être choisis parmi les polymères amphiphiles statistiques d'AMPS modifiés par réaction avec une n-monoalkylamine ou une di-n-alkylamine en C₆-C₂₂ tels que ceux décrits dans la demande de brevet WO-A-00/31154 (faisant partie intégrante du contenu de la description). Ces polymères peuvent également contenir d'autres monomères hydrophiles à insaturation éthylénique choisis par exemple parmi l'acide acrylique, l'acide méthacrylique ou leurs dérivés alkyl substitués en β ou leurs esters obtenus avec des mono- ou poly-alkylèneglycols, l'acrylamide, le méthacrylamide, l'acide itaconique, l'acide styrène sulfonique, l'acide vinylsulfonique, l'acide (meth)allylsulfonique, l'acide vinylphosphonique, la N-vinylacétamide, la N-méthyl N-vinylacétamide, la N-vinylformamide, la N-méthyl N-vinylformamide, les N-vinyllactames comportant un groupe alkyle ayant de 4 à 9 atomes de carbone, tels que N-vinylpyrrolidone, la N-butyrolactame et la N-vinylcaprolactame, l'anhydride maléique, l'acrylonitrile, le (meth)acrylate de glycidyle, le chlorure de vinyle, le chlorure de vinylidène, le chlorure de méthyldiallylammonium, le méthacrylate de diméthylaminoéthyle quaternisé (MADAME), le chlorure de (méth)acrylamidopropyl-triméthylammonium (APTAC et MAPTAC), le chlorure de méthylvinylimidazolium, ou leurs mélanges.

Les copolymères préférentiels de l'invention sont choisis parmi les copolymères amphiphiles d'AMPS et d'au moins un monomère à insaturation éthylénique comportant au moins une partie hydrophobe ayant de 6 à 30 atomes de carbone et préférentiellement de 6 à 22 atomes de carbone et plus préférentiellement de 6 à 18 atomes de carbone et plus particulièrement de 12 à 18 atomes de carbone.

Ces mêmes copolymères peuvent contenir en plus un ou plusieurs comonomères hydrophiles à insaturation éthylénique comme l'acide acrylique, l'acide méthacrylique ou leurs dérivés alkylsubstitués en β ou leurs esters obtenus avec des mono- ou poly-alkylèneglycols, l'acrylamide, le méthacrylamide, l'acide itaconique, l'acide styrène sulfonique, l'acide vinylsulfonique, l'acide (meth)allylsulfonique, l'acide vinylphosphonique, la N-vinylacétamide, la N-méthyl N-vinylacétamide, la N-vinylformamide, la N-méthyl N-vinylformamide, les N-vinyllactames comportant un groupe alkyle ayant de 4 à 9 atomes de carbone, tels que N-vinylpyrrolidone, la N-butyrolactame et la N-vinylcaprolactame, l'anhydride maléique, l'acrylonitrile, le (meth)acrylate de glycidyle, le chlorure de vinyle, le chlorure de vinylidène, le chlorure de méthyldiallylammonium, le méthacrylate de diméthylaminoéthyle quaternisé (MADAME), le chlorure de (méth)acrylamidopropyl-triméthylammonium (APTAC et MAPTAC), le chlorure de méthylvinylimidazolium et leurs mélanges.

Les monomères hydrophobes à insaturation éthylénique de ces copolymères particuliers sont choisis de préférence parmi les acrylates ou les acrylamides de formule (1) suivante : 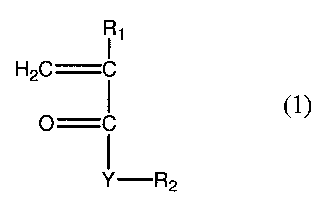 dans laquelle R₁ désigne un atome d'hydrogène, un radical alkyle linéaire ou ramifié en C₁-C₆ (de préférence méthyle) ; Y désigne O ou NH ou NH-CO-O; R₂ désigne un radical hydrophobe hydrocarboné ayant de 6 à 22 atomes de carbone, et de préférence de 6 à 18, et plus particulièrement de 12 à 18 atomes de carbone.

Le radical hydrophobe R₂ est choisi de préférence parmi les radicaux alkyles et alcényles en C₆-C₁₈, linéaires (par exemple n-hexyle, n-octyle, n-décyle, n-hexadécyle, n-dodécyle, oléyle), ramifiés (par exemple isostéarique) ou alicycliques (par exemple cyclododécane ou adamantane), les radicaux perfluoroalkyles en C₆-C₁₈, par exemple le groupement de formule : 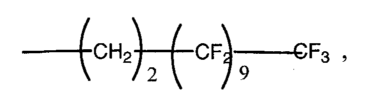 le radical cholestéryle ou un ester de cholestérol comme l'hexanoate de cholestéryle ; les groupes polycycliques aromatiques comme le naphtalène ou le pyrène. Parmi ces radicaux, on préfère plus particulièrement les radicaux alkyles linéaires et ramifiés.

Selon une forme particulièrement préférée de l'invention, le radical hydrophobe R₂ comporte en plus au moins un motif oxyde d'alkylène et de préférence une chaîne polyoxyalkylénée. La chaîne polyoxyalkylénée de façon préférentielle est constituée de motifs oxyde d'éthylène et/ou de motifs oxyde de propylène et encore plus particulièrement constituée uniquement de motifs oxyde d'éthylène. Le nombre de moles de motifs oxyalkylénés varie en général de 1 à 100 moles et préférentiellement de 1 à 30 moles et plus préférentiellement de 3 à 20 moles.

Parmi ces copolymères, on peut citer les copolymères comportant de 0 à 45% en moles de motif acrylamide, de 5 à 50% en moles de motif AMPS partiellement ou totalement neutralisé et comportant au moins 50% en moles de motifs portant une chaîne n-(C₆-₁₈)alkyl ou alkyl polyoxyéthylénée, de préférence au moins 60% et encore mieux au moins 65%.

On citera plus particulièrement les copolymères constitués :
(a) de 5 à 50 % en moles de motif acide 2-acrylamido-2-méthylpropane sulfonique (AMPS) de formule (2) suivante : 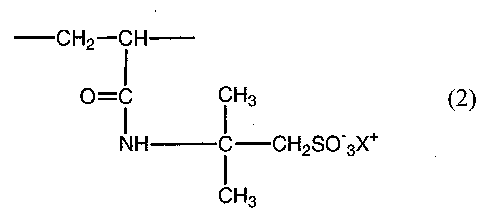 dans laquelle X⁺ est un proton, un cation de métal alcalin, un cation alcalino-terreux ou l'ion ammonium ou l'ion ammonium substitué par un groupe alkyle portant de 1 à 15 atomes de carbone ; et
(b) de 50 à 95% en moles de motif de formule (3) suivante : 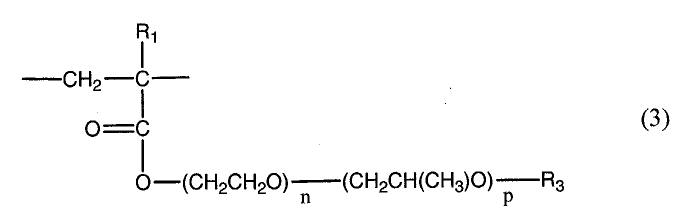
dans laquelle n et p, indépendamment l'un de l'autre désignent un nombre de moles et varie de 0 à 100, de préférence de 1 à 30 et plus préférentiellement de 3 à 20 sous réserve que n+p soit inférieur ou égal à 100, de préférence inférieur à 30 et encore mieux inférieur à 20 ; R₁ a la même signification indiquée ci-dessus pour la formule (1) et R₃ désigne un alkyle linéaire ou ramifié comportant de 6 à 22 atomes de carbone, de préférence de 6 à 18 atomes de carbone et encore mieux de 12 à 18 atomes de carbone.

La quantité stabilisatrice de polymère ou copolymère est toute quantité suffisante pour conférer à l'émulsion E/H la stabilité requise, en particulier suffisante pour éviter le phénomène de floculation de l'émulsion (déstabilisation).

Les polymères et copolymères conformes à l'invention sont généralement présents dans les compositions dans des concentrations massiques allant de 0,1 à 10 %, préférentiellement de 0,5 à 8 % et mieux de 1 à 5 % par rapport au poids total de la composition.

Le caractère non floculé des émulsions peut être mis en évidence par microscopie optique, après dilution si nécessaire lorsque les émulsions sont concentrées. Une émulsion non floculée est constituée de gouttes indépendantes les unes des autres ; une émulsion floculée comporte des agrégats de gouttes, ce qui n'est pas observé dans les compositions de l'invention.

La phase grasse ou phase huileuse contient habituellement au moins une huile. Comme huiles utilisables dans la composition de l'invention, on peut citer par exemple :
- les huiles hydrocarbonées d'origine animale, telles que le perhydrosqualène ;
- Les huiles hydrocarbonées d'origine végétale, telles que les triglycérides liquides d'acides gras comportant de 4 à 22 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société STEARINERIES DUBOIS ou ceux vendus sous les dénominations MIGLYOL 810, 812 et 818 par la société DYNAMIT NOBEL, l'huile de jojoba, l'huile de beurre de karité ;
- Les esters et les éthers de synthèse, notamment d'acides gras, comme les huiles de formules R'COOR" et R'OR" dans laquelle R' représente le reste d'un acide gras comportant de 8 à 29 atomes de carbone, et R" représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérylthrityle ;
- Les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam® ;
- Les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- Les alcools gras alcoylés et les acides gras alcoylés, et notamment éthoxylés, tels que l'oleth-12 et le Stéarate de PEG-20 ;
- Les huiles fluorées partiellement hydrocarbonées et/ou siliconées comme celles décrites dans le document JP-A-2.295912. Comme huiles fluorées, on peut citer aussi le perfluorométhylcyclopentane et le perfluoro-1,3 diméthylcyclohexane, vendus sous les dénominations de « FLUTEC PC1® » et « FLUTEC PC3® » par la société BNFL Fluorochemicals ; le perfluoro-1,2-diméthylcyclobutane; les perfluoroalcanes tels que le dodécafluoropentane et le tétradécafluorohexane, vendus sous les dénominations de « PF 5050® » et « PF 5060® » par la société 3M, ou encore le bromoperflurooctyle vendu sous la dénomination « FORALKYL® » par la société ATOCHEM ; le nonafluorométhoxybutane vendu sous la dénomination « MSX 4518® » par la société 3M et le nonafluoroéthoxyisobutane ; les dérivés de perfluoromorpholine, tels que la 4-trifluorométhyl perfluoromorpholine vendue sous la dénomination « PF 5052® » par la société 3M ;
- Les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendants ou en bout de chaîne siliconée, groupements ayant de 2 à 24 atomes de carbone; les silicones phénylées comme les phényltriméthicones, les phényldiméthicones, les phényltriméthylsiloxydiphényl-siloxanes, les diphényl-diméthicones, les diphénylméthyl-diphényl trisiloxanes, les 2-phényléthyltriméthyl-siloxysilicates, et les leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluorés, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch; les gommes telles que les gommes de silicone (diméthiconol) ; les résines de silicone telles que la trifluorométhyl-C₁₋₄-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société DOW CORNING ou sous les dénominations « GRANSIL » par la société GRANT INDUSTRIES, ainsi que les autres gélifiants de la phase huileuse habituellement utilisés.

Ces corps gras peuvent être choisis de manière variée par l'homme du métier afin de préparer une composition ayant les propriétés, par exemple de consistance ou de texture, souhaitées.

La quantité de phase huileuse dans l'émulsion de l'invention peut aller par exemple de 2 à 60 %, de préférence de 5 à 50 % et mieux de 10 à 40 % en poids par rapport au poids total de la composition.

De façon connue, la composition de l'invention peut contenir également des adjuvants habituels dans le domaine cosmétique et/ou dermatologique, autres que ceux cités précédemment, tels que les solvants, les actifs, les conservateurs, les antioxydants, les agents complexants, les parfums, les filtres UV organiques ou minéraux, les sels, les charges, les bactéricides, les absorbeurs d'odeur, les matières colorantes et encore les vésicules lipidiques. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse et/ou dans les vésicules lipidiques. Bien entendu, l'homme du métier veillera à choisir les éventuels additifs complémentaires et/ou leur quantité de telle manière que les propriétés avantageuses de la composition selon l'invention ne soient pas ou substantiellement pas altérées par l'adjonction envisagée.

Comme actifs utilisables dans la composition de l'invention, on peut citer par exemple les agents hydratants et par exemple les hydrolysats de protéines et les polyols tels que la glycérine, les glycols comme les polyéthylène glycols, et les dérivés de sucre; les extraits naturels; les oligomères procyannidoliques ; les vitamines comme la vitamine A (rétinol), la vitamine C (acide ascorbique), la vitamine E (tocophérol), la vitamine B5 (panthénol), la vitamine B3 (niacinamide) ; l'urée; la caféine; les dépigmentants tels que l'acide kojique et l'acide caféique ; l'acide salicylique ; les alpha-hydroxyacides tels que l'acide lactique et l'acide glycolique ; les rétinoïdes tels que les caroténoïdes ; l'hydrocortisone ; la mélatonine ; les extraits d'algues, de champignons, de végétaux, de levures, de bactéries ; les protéines hydrolysées, partiellement hydrolysées ou non hydrolysées, les enzymes ; la DHEA et ses dérivés et métabolites ; les actifs anti-bactériens pour le traitement des peaux grasses comme le 2,4,4'-trichloro-2'-hydroxy diphényl éther (ou triclosan), le 3,4,4'-trichlorocarbanilide (ou triclocarban) ; les agents matifiants comme les fibres ; les filtres solaires (filtres U.V.), et leurs mélanges.

Comme filtres UV chimiques, on peut citer par exemple :
- le Butylmethoxydibenzoylmethane vendu notamment par la société HOFFMANN LA ROCHE sous la dénomination PARSOL 1789,
- l'octocrylène vendu notamment par la société BASF sous la dénomination UVINUL N539,
- l'octylsalicylate vendu notamment par la société Haarman-Reimer sous la dénomination Neo Heliopan OS,
- l'octylméthoxycinnamate vendu notamment par la société Hoffmann Laroche sous la dénomination PARSOL MCX,
- l'acide phénylbenzimidazole sulfonique vendu notamment par la société Merck sous la dénomination Eusolex 232,
- les oxybenzones telles les benzophénones-3, -4 ou -5,
- les silicones benzotriazoles et en particulier le drométrizole trisiloxane, et ceux décrits dans la demande FR-A-2,642,968,
- l'acide téréphtalylidène di-camphre sulfonique, en particulier le Mexoryl SX vendu par la société Chimex,
- ainsi que les filtres organiques insolubles comme les dérivés de triazine, et en particulier la 2,4-bis ([4-(2-éthyl-hexyloxy)-2-hydroxy]-phényl)-6-(4-méthoxy-phényl)-1,3,5-triazine disponible auprès de la société CIBA GEIGY sous la dénomination TINOSORB S, et le 2,2'-méthylènebis-[6-(2H benzotriazol-2-yl)4-(1,1,3,3-tétraméthylbutyl)-phénol] disponible auprès de la société CIBA GEIGY sous la dénomination TINOSORB M.

Comme filtres U.V. physiques, on peut citer par exemple les oxydes de titane ou de zinc, sous forme de micro- ou nanoparticules (nanopigments) éventuellement enrobées.

Le ou les actifs peuvent être par exemple présents en une concentration allant de 0,01 à 20 %, de préférence de 0,1 à 10 % et mieux de 0,5 à 5 % du poids total de la composition.

Comme charges, on peut citer par exemple, outre les pigments, la poudre de silice ; le talc ; les particules de polyamide et notamment celles vendues sous la dénomination ORGASOL par la société Atochem ; les poudres de polyéthylène; les microsphères à base de copolymères acryliques, telles que celles en copolymère diméthacrylate d'éthylène glycol/methacrylate de lauryle vendues par la société Dow Corning sous la dénomination de POLYTRAP; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; les latex, et leurs mélanges. Les charges peuvent être par exemple présentes en une concentration allant de 0,01 à 20 %, de préférence de 0,1 à 10 % et mieux de 0,5 à 5 % du poids total de la composition.

La composition de l'invention peut éventuellement contenir des tensioactifs, par exemple des tensioactifs nettoyants (qui sont des tensioactifs moussants et non des tensioactifs émulsionnants) et notamment des tensioactifs non ioniques tels que les alcools ou acides gras éthoxylés ou glycérolés; des tensioactifs anioniques comme les alkyléther sulfonates ou les alkyl éther sulfates ou les ester et éther phosphates ; des tensioactifs cationiques comme les sels d'acide gras cationiques tels que les Esterquats; des tensioactifs amphotères et zwitterioniques comme les alkylbétaïnes,; et leurs mélanges.

La composition de l'invention peut contenir dans la phase aqueuse, un ou plusieurs polymères hydrophiles choisis par exemple parmi les polymères carboxyvinyliques tels que les carbopols (carbomers) ; les polymères d'acide 2-acrylamido-2-méthylpropane sulfonique solubles ou dispersibles en phase aqueuse comme le polymère commercialisé sous la dénomination « Hostacerin AMPS » (nom CTFA : ammonium polyacryldimethyltauramide) par la société Clariant ; les polymères neutres synthétiques comme le polyvinylpyrrolidone (PVP), l'acétate de polyvinyle (PVA) ; les polysaccharides comme les gommes de guar, de xanthane et les dérivés de cellulose ; les dérivés siliconés hydrosolubles ou hydrodispersibles comme les silicones acryliques et les silicones cationiques ; et leurs mélanges. Le ou les polymères peuvent être par exemple présents en une concentration allant de 0,01 à 20%, de préférence de 0,05 à 10% et mieux de 0,1 à 5% du poids total de la composition.

La composition selon l'invention se présente sous forme d'émulsions eau dans huile de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

De manière avantageuse, selon un mode particulier de réalisation de l'invention, les émulsions préparées avec les polymères et/ou copolymères selon l'invention peuvent comporter seulement 1% en poids ou moins, et même être exemptes de tensioactifs émulsionnants, tout en étant stables au stockage.

La composition selon l'invention peut être utilisée dans beaucoup d'applications cosmétiques ou dermatologiques, notamment elle peut être utilisée pour le traitement, le soin, le maquillage et/ou le nettoyage de la peau du visage et/ou du corps, des muqueuses (lèvres), du cuir chevelu et/ou des fibres kératiniques (cheveux ou cils), par exemple pour donner au visage un teint lumineux et éclatant, une bonne mine, un aspect lisse et plus jeune, pour traiter les rides et ridules de la peau, pour dépigmenter la peau et notamment enlever les taches de vieillissement, pour faire disparaître les comédons, pour traiter les cheveux abîmés, les tonifier, leur redonner de la vigueur, et renforcer les fibres kératiniques.

Ainsi, les compositions de l'invention peuvent être utilisées comme produit de soin et/ou d'hygiène, tels que des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage (y compris le démaquillage) ou le gommage de la peau. Elles peuvent constituer aussi des produits pour le maquillage des fibres kératiniques, de la peau, des lèvres et/ou des ongles. Elles peuvent notamment constituer un fond de teint, un fard à joues ou à paupières, un rouge à lèvres, un mascara ou un eye-liner.

Les compositions de l'invention peuvent être également utilisées comme produit solaire pour protéger la peau des rayons U.V.

Les compositions selon l'invention peuvent être utilisées comme produits capillaires rincés ou non-rincés, notamment pour le lavage, le soin, le conditionnement, le maintien de la coiffure ou la mise en forme des fibres kératiniques telles que les cheveux. Elles peuvent également consister en des préparations solides constituant des savons ou des pains de nettoyage.

Les compositions de l'invention peuvent être également utilisées comme produit de soin bucco-dentaire tel que des pâtes dentifrices.

Elles peuvent être aussi des produits de coiffage tels que des lotions de mise en plis, des lotions pour le brushing, des compositions de fixation et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une mousse pour la fixation ou le traitement des cheveux.

Ainsi, la présente invention a également pour objet l'utilisation cosmétique de la composition selon l'invention, pour le traitement, le soin, le maquillage et /ou le nettoyage de la peau du visage et/ou du corps, des muqueuses (lèvres), du cuir chevelu et/ou des fibres kératiniques.

Un autre objet de l'invention est un procédé de traitement cosmétique des matières kératiniques humaines comme la peau, y compris le cuir chevelu, les cheveux, les cils, des sourcils, les ongles ou les muqueuses notamment les lèvres, caractérisé par le fait qu'on applique sur les matières kératiniques, une composition cosmétique telle que définie ci-dessus, selon la technique d'utilisation habituelle de cette composition. Par exemple, application de crèmes, de gels, de sérums, de lotions, de laits sur la peau, le cuir chevelu et/ou les muqueuses. Le type de traitement est fonction du ou des actifs présents dans la composition.

L'invention a encore pour objet une utilisation de la composition ci-dessus pour préparer une pommade ou un onguent destiné à traiter le visage et/ou le corps humain, y compris les mains, notamment pour traiter l'acné.

Les exemples suivants illustrent la présente invention. Dans les exemples, sauf indication contraire, tous les pourcentages et parties sont exprimés en poids.

### Exemple 1 - Crème de soin

Dans cet exemple, on utilise un copolymère AMPS comportant des greffons alkyl(C₁₂-₁₄) à 3 unités oxyéthylénées (taux de greffage molaire = 69%) dont la solution à 1% dans un mélange d'acétate de tocophéryl (13,3% en poids) et de triglycérides caprylique/caprique C₈-₁₀ 60/40 (86,7%) présente une transmittance de la lumière, à une longueur d'onde de 500 nm, égale à 68%. Cette mesure de transmittance est effectuée à l'aide d'un spectrophotomètre Cary 300 (Varian), en utilisant des cuves d'épaisseur 1 cm.

| | |
|---|---|
| Phase huileuse | % |
| Copolymère AMPS comportant des greffons C₁₂-₁₄, 3 unités oxyéthylénées (taux de greffage molaire = 69%) | 2,5 |
| Tocophéryl acétate | 3 |
| Caprylique/caprique triglycérides C₈-₁₀ 60/40 | 19,5 |

| | |
|---|---|
| Phase aqueuse | % |
| Glycérine | 3 |
| Sulfate de magnésium | 2,25 |
| Conservateur | 0,07 |
| Eau déminéralisée | 69,68 |

### Mode de préparation

Le copolymère amphiphile d'AMPS est solubilisé pendant 48 heures sous agitation dans la phase huileuse à 50°C ; la solution obtenue est macroscopiquement homogène. L'émulsion est préparée par introduction lente de la phase aqueuse dans la phase huileuse sous agitation à l'aide d'un homogénéisateur de type Moritz sous une vitesse d'agitation de 1500 tours/minute.

La taille des gouttes de l'émulsion obtenue est comprise entre 100 nm et 2 microns. La viscosité sous une vitesse de cisaillement de 200 s⁻¹ est égale à 1,4 Pa.s (mesurée à l'aide d'un rhéomètre Haake RS150 équipé d'une géométrie cône plan 6cm/2° à environ 25°C).

Les figures 1 et 2 sont des photographies de l'émulsion eau dans huile obtenue faites à l'aide d'un microscope optique en mode contraste interférentiel, aux grossissements X 400 (figure 1) et X 1000 (figure 2).

Comme le montrent les figures 1 et 2, l'émulsion n'est pas floculée et présente une belle texture de type crème, peu grasse et agréable à appliquer.

### Exemple 2 - Lait pour peau sèche

Dans cet exemple, on utilise un copolymère AMPS comportant des greffons C₁₂-₁₄, 3 unités oxyéthylénées (taux de greffage molaire = 69%) dont la solution à 1% dans un mélange d'acétate de tocophéryl (13,3% en poids) et de triglycérides caprylique/caprique C₈-₁₀ 60/40 (86,7%) présente une transmittance de la lumière, à une longueur d'onde de 500 nm, égale à 68%. Cette mesure de transmittance est effectuée à l'aide d'un spectrophotomètre Cary 300 (Varian), en utilisant des cuves d'épaisseur 1 cm.

| | |
|---|---|
| Phase huileuse | % |
| Copolymère AMPS comportant des greffons C₁₂-₁₄, 3 unités oxyéthylénées (taux de greffage molaire = 69%) | 2,5 |
| Tocophéryl acétate | 3 |
| Caprylique/caprique triglycérides C₈-₁₀ 60/40 | 25,5 |
| Gélifiant huileux | 4 |

| | |
|---|---|
| Phase aqueuse | % |
| Glycérine | 3 |
| Sulfate de magnésium | 1,95 |
| Conservateur | 0,06 |
| Eau déminéralisée | 59,99 |

### Mode de préparation

Le copolymère amphiphile d'AMPS est solubilisé pendant 48 heures sous agitation dans la phase huileuse à 50°C ; la solution obtenue est macroscopiquement homogène. L'émulsion est préparée par introduction lente de la phase aqueuse dans la phase huileuse sous agitation à l'aide d'un homogénéisateur de type Mortiz sous une vitesse d'agitation de 1500 tours/minute.

La taille des gouttes est de l'ordre du micron. La viscosité sous une vitesse de cisaillement de 200 s⁻¹ est égale à 0,6 Pa.s (mesurée à l'aide d'un rhéomètre Haake RS150 équipé d'une géométrie cône plan 6cm/2° à environ 25°C).

L'émulsion n'est pas floculée et présente une belle texture fluide adaptée à un lait pour le corps, de type peau sèche.

### Exemple comparatif

Dans ce contre-exemple, on utilise un copolymère AMPS comportant des greffons isostéariques, 20 unités oxyéthylénées (taux de greffage molaire = 40,8%) dont la solution à 1% dans un mélange d'acétate de tocophéryl (13,3% en poids) et de triglycérides caprylique/caprique C₈-₁₀ 60/40 (86,7%) présente une transmittance de la lumière, à une longueur d'onde de 500 nm, égale à 2%. Cette mesure de transmittance est effectuée à l'aide d'un spectrophotomètre Cary 300 (Varian), en utilisant des cuves d'épaisseur 1 cm.

| Phase huileuse | % |
|---|---|
| Copolymère AMPS comportant des greffons isostéarique, 20 unités oxyéthylénés (taux de greffage molaire = 40,8%) | 2,5 |
| Tocophéryl acétate | 3 |
| Caprylique/caprique triglycérides C₈-₁₀ 60/40 | 24,5 |
| Gélifiant huileux | 4 |

| Phase aqueuse | % |
|---|---|
| Glycérine | 3 |
| Conservateur | 0,07 |
| Eau déminéralisée | 66,93 |

### Mode de préparation

Le copolymère amphiphile d'AMPS est introduit pendant 48 heures sous agitation dans la phase huileuse à 50°C ; la solution obtenue contient des agrégats polymériques non solubilisés, observables visuellement. Après 24 heures au repos, cette solutions contient des agrégats de polymère sédimentés. L'émulsion est préparée par introduction lente de la phase aqueuse dans la phase huileuse sous agitation à l'aide d'un homogénéisateur de type Moritz sous une vitesse d'agitation de 1500 tours/minute.

L'émulsion obtenue est de type huile dans eau, et non eau dans huile comme dans les exemples selon l'invention.

## Revendications

1. Composition cosmétique et/ou dermatologique sous forme d'une émulsion eau-dans-huile comprenant une phase aqueuse dispersée dans une phase huileuse, **caractérisée en ce qu'**elle comprend une quantité stabilisatrice de la phase aqueuse d'au moins un polymère ou copolymère amphiphile, de l'acide 2-acrylamido-2-méthylpropane sulfonique portant des greffons hydrophobes choisis parmi les radicaux hydrocarbonés ayant de 6 à 30 atomes de carbone, les radicaux hydrocarbonés oxyalkylénés comprenant un groupe hydrocarboné ayant de 6 à 30 atomes de carbone et au moins un motif oxyalkylène, les radicaux siliconés, les radicaux perfluoroalkyles et leurs mélanges, le taux de greffage molaire en motif hydrophobe du polymère ou copolymère étant au moins égal à 50 %.

2. Composition selon la revendication 1, **caractérisée en ce que** le polymère ou copolymère est liposoluble ou lipodispersible, ou soluble ou dispersible dans un solvant polaire ayant une constante diélectrique mesurée à 25°C, inférieure à 50.

3. Composition selon la revendication 1 ou 2, **caractérisée en ce que** le taux de greffage molaire du polymère ou copolymère est égal ou supérieur à 60 %, de préférence égal ou supérieur à 65 %.

4. Composition selon l'une quelconque des revendications 1 à 3, **caractérisée en ce que** les radicaux hydrocarbonés des greffons hydrophobes comprennent de 6 à 22 atomes de carbone, de préférence 6 à 18 atomes de carbone et mieux 12 à 18 atomes de carbone.

5. Composition selon la revendication 4, **caractérisée en ce que** les radicaux hydrocarbonés sont choisis parmi les radicaux alkyles linéaires ou ramifiés, alcènyles linéaires ou ramifiés, alicycliques, le radical cholestéryle et les restes d'esters de cholestérol, et les radicaux polycycliques aromatiques.

6. Composition selon l'une quelconque des revendications 1 à 3, **caractérisée en ce que** les radicaux hydrocarbonés oxyalkylénés comprennent un groupe hydrocarboné ayant 6 à 22 atomes de carbone, de préférence 6 à 18 atomes de carbone et mieux 12 à 18 atomes de carbone, et 1 à 100, de préférence 1 à 30 et mieux 3 à 20 motifs oxyalkylène.

7. Composition selon la revendication 6, **caractérisée en ce que** les radicaux hydrocarbonés oxyalkylénés répondent à la formule : 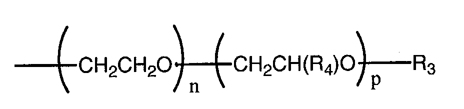 dans laquelle R₃ représente un radical hydrocarboné en C₆-C₃₀, R₄ représente l'hydrogène ou un groupe alkyle linéaire ou ramifié en C₁-C₆ (de préférence méthyle) et n et p sont, indépendamment l'un de l'autre, des entiers de 0 à 100, de préférence 1 à 30, mieux de 3 à 20 sous réserve que la somme n+p, non nulle, soit égale ou inférieure à 100, de préférence inférieure à 30 et mieux inférieure à 20.

8. Composition selon l'une quelconque des revendications précédentes, **caractérisée en ce que** les greffons hydrophobes sont liés de manière covalente à la chaîne principale du polymère ou du copolymère, de préférence par l'intermédiaire d'un groupe de liaison fonctionnel choisi parmi les groupes ester, amide, éther ou uréthane.

9. Composition selon la revendication 1, **caractérisée en ce que** le copolymère amphiphile à greffons hydrophobes est un copolymère de l'acide 2-acrylamido-2-méthylpropane sulfonique et d'au moins un monomère à insaturation éthylénique comportant une partie hydrophobe choisi parmi les radicaux hydrocarbonés ayant de 6 à 30 atomes de carbone, de préférence 6 à 22 atomes de carbone, mieux 6 à 18 atomes de carbone et mieux encore 12 à 18 atomes de carbone, les radicaux hydrocarbonés oxyalkylénés comportant un radical hydrocarboné tel que défini ci-dessus, les radicaux perfluororalkyles et les radicaux siliconés, et au moins un motif oxyalkylène.

10. Composition selon la revendication 9, **caractérisée en ce que** le monomère hydrophobe à insaturation éthylénique répond à la formule : 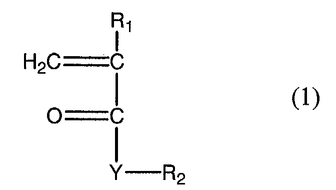 dans laquelle R₁ désigne un atome d'hydrogène, un radical alkyle linéaire ou ramifié en C₁-C₆ (de préférence méthyle) ; Y désigne O ou NH ou NH-CO-O; R₂ désigne un radical hydrophobe hydrocarboné ayant de 6 à 22 atomes de carbone, et de préférence de 6 à 18, et plus particulièrement de 12 à 18 atomes de carbone
et/ou un radical hydrocarboné tel que défini précédemment comportant en plus 1 à 100 moles, de préférence 1 à 30 et mieux 3 à 20 moles de motifs oxyalkylénés.

11. Composition selon la revendication 9, **caractérisée en ce que** le copolymère amphiphile est également un copolymère d'au moins un autre comonomère hydrophile à insaturation éthylénique.

12. Composition selon la revendication 11, **caractérisée en ce que** le comonomère hydrophile est choisi parmi l'acide acrylique, l'acide méthacrylique ou leurs dérivés alkylsubstitués en β ou leurs esters obtenus avec des mono- ou poly-alkylèneglycols, l'acrylamide, le méthacrylamide, l'acide itaconique, l'acide styrène sulfonique, l'acide vinylsulfonique, l'acide (meth)allylsulfonique, l'acide vinylphosphonique, la N-vinylacétamide, la N-méthyl N-vinylacétamide, la N-vinylformamide, la N-méthyl N-vinylformamide, les N-vinyllactames comportant un groupe alkyle ayant de 4 à 9 atomes de carbone, l'anhydride maléique, l'acrylonitrile, le (meth)acrylate de glycidyle, le chlorure de vinyle, le chlorure de vinylidène, le chlorure de méthyldiallylammonium, le méthacrylate de diméthylaminoéthyle quaternisé, le chlorure de (méth)acrylamidopropyl-triméthylammonium, le chlorure de méthylvinylimidazolium et leurs mélanges.

13. Composition selon la revendication 1, **caractérisée en ce que** le copolymère amphiphile à greffons hydrophobes comprend :
- 0 à 45 % en moles de motifs acrylamide ;
- 5 à 50 % en moles de motifs AMPS partiellement ou totalement neutralisés ; et
- 50 % ou plus, de préférence 60 % ou plus, et mieux 65 % ou plus, en moles de motifs n(C₆-C₁₈) alkyle ou alkyl polyoxyéthylénés.

14. Composition selon la revendication 1, **caractérisée en ce que** le copolymère amphiphile à greffons hydrophobes est constitué de :
(a) 5 à 50 % en moles de motif acide 2-acrylamido-2-méthylpropane sulfonique (AMPS) de formule (2) suivante : 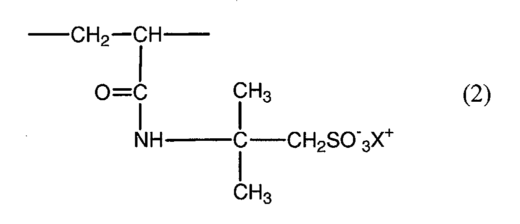 dans laquelle X⁺ est un proton, un cation de métal alcalin, un cation alcalino-terreux ou l'ion ammonium ou l'ion ammonium substitué par un groupe alkyle portant de 1 à 15 atomes de carbone ; et
(b) 50 à 95 % en moles de motif de formule (3) suivante : 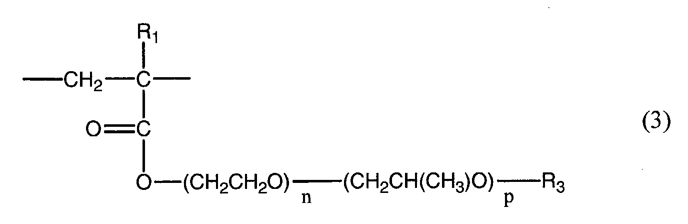
dans laquelle n et p, indépendamment l'un de l'autre désignent un nombre de moles et varie de 0 à 100, de préférence de 1 à 30 et plus préférentiellement de 3 à 20 sous réserve que n+p soit inférieur ou égal à 100, de préférence inférieur à 30 et encore mieux inférieur à 20 ; R₁ est un atome d'hydrogène ou un radical alkyle, linéaire ou ramifié, en C₁-C₆ et R₃ désigne un alkyle linéaire ou ramifié comportant de 6 à 22 atomes de carbone, de préférence de 6 à 18 atomes de carbone et encore mieux de 12 à 18 atomes de carbone.

15. Composition selon l'une quelconque des revendications précédentes **caractérisée en ce que** le polymère ou copolymère amphiphile est sous forme libre ou partiellement ou totalement neutralisé.

16. Composition selon l'une quelconque des revendications précédentes, **caractérisée en ce que** le polymère ou copolymère amphiphile a une masse molaire moyenne en poids de 50.000 à 10.000.000 g/mole, de préférence 100.000 à 8.000.000 g/mole et mieux de 100.000 à 7.000.000 g/mole.

17. Composition selon l'une quelconque des revendications précédentes, **caractérisée en ce que** le polymère ou copolymère amphiphile n'est pas réticulé.

18. Composition selon l'une quelconque des revendications précédentes, **caractérisée en ce que** la quantité stabilisatrice de polymère ou copolymère représente 0,1 à 10 %, de préférence 0,5 à 8 % et mieux 1 à 5 % en poids par rapport au poids total de la composition.

19. Composition selon l'une quelconque des revendications précédentes, **caractérisée en ce qu'**elle renferme 1% en poids ou moins de tensioactifs émulsionnants par rapport au poids total de la composition.

20. Composition selon l'une quelconque des revendications 1 à 18, **caractérisée en ce qu'**elle ne contient pas de tensioactifs émulsionnants.

21. Composition selon l'une quelconque des revendications précédentes, **caractérisée en ce que** la phase huileuse représente 2 à 60%, de préférence 5 à 50%, et mieux 10 à 40% en poids par rapport au poids total de la composition.

22. Composition selon l'une quelconque des revendications précédentes, **caractérisée en ce que** la phase huileuse comprend au moins 5% en poids d'huiles hydrocarbonées par rapport au poids total de la composition.

23. Utilisation dans une composition cosmétique et/ou dermatologique, sous forme d'une émulsion eau dans huile comprenant une phase aqueuse dispersée dans une phase huileuse, en tant qu'agent de stabilisation de la phase aqueuse, d'au moins un polymère ou copolymère amphiphile, de l'acide 2-acrylamido-2-méthylpropane sulfonique portant des greffons hydrophobes choisis parmi les radicaux hydrocarbonés ayant de 6 à 30 atomes de carbone, les radicaux oxyalkylénés comprenant un groupe hydrocarboné en C₆-C₃₀ et au moins un motif oxyalkylène, les radicaux siliconés, les radicaux perfluoroalkyles, et leurs mélanges, le taux de greffage molaire en motif hydrophobe du polymère ou copolymère étant au moins égal à 50%.

24. Utilisation selon la revendication 23, **caractérisée en ce que** le polymère ou copolymère est conforme à l'une quelconque des revendications 2 à 17.

25. Utilisation selon la revendication 23 ou 24, **caractérisée en ce que** la quantité stabilisatrice de polymère ou copolymère représente 0,1 à 10%, de préférence 0,5 à 8% et mieux 1 à 5% en poids par rapport au poids total de la composition.

26. Utilisation selon l'une quelconque des revendications 23 à 25, **caractérisée en ce qu'**elle renferme 1% en poids au moins de tensioactifs émulsionnants par rapport au poids total de la composition.

27. Utilisation selon l'une quelconque des revendications 23 à 25, **caractérisée en ce qu'**elle ne contient pas de tensioactifs émulsionnants.

28. Utilisation selon l'une quelconque des revendications 23 à 27, **caractérisée en ce que** la phase huileuse représente 2 à 60%, de préférence 5 à 50%, et mieux 10 à 40% en poids par rapport au poids total de la composition.

29. Utilisation selon l'une quelconque des revendications 23 à 28, **caractérisée en ce que** la phase huileuse comprend au moins 5% en poids d'huiles hydrocarbonées par rapport au poids total de la composition.

30. Utilisation cosmétique pour le traitement, le soin, le maquillage ou le nettoyage de la peau du visage et/ou du corps, des muqueuses, du cuir chevelu et/ou des fibres kératiniques d'une composition cosmétique selon l'une quelconque des revendications 1 à 22.

31. Procédé de traitement cosmétique des matières kératiniques, **caractérisé par le fait qu'**on applique sur les matières kératiniques, une composition cosmétique selon l'une quelconque des revendications 1 à 22.

32. Procédé selon la revendication précédente, **caractérisé en ce que** la matière kératinique est la peau.
